# EUROPEAN PATENT APPLICATION

(11) **EP 3 415 910 A1**
(43) Date of publication of application: **19.12.2018**
(21) Application number: 17176436.8
(22) Date of filing: 16.06.2017
(51) Int. Cl.: G01N 33/543, G01N 33/558, G01N 33/58

(54) **A METHOD OF QUANTITATIVE DETERMINATION OF SARCOSINE IN A BIOLOGICAL SAMPLE USING ANTI-ARCOSINE ANTIBODIES AND PEROXIDASE-ACTIVE GOLD NANOPARTICLES OR QUANTUM DOTS**

(71) Applicant: Prevention Medicals s.r.o., 742 13 Studenka - Butovice (CZ)
(72) Inventor: UHLIROVA, Dagmar, 602 00 Brno (CZ); DOCEKALOVA, Michaela, 696 11 Mutenice (CZ); STANKOVA, Martina, 664 34 Kurim (CZ); MELICHAR, Lukas, 691 52 Kostice (CZ); RUZICKA, Josef, 796 01 Prostejov (CZ); KIZEK, Rene, 679 21 Cerna Hora (CZ)
(74) Representative: Rausch Wanischeck-Bergmann Brinkmann

(57) **Abstract**

The invention deals with a method of quantitative determination of sarcosine in a biological sample with a sensitivity from 0.05 µM to 1.00 µM by means of anti-sarcosine antibodies and peroxidase-active gold nanoparticles or quantum dots, applying the ELISA and LFIA methods. It is based on the usage of immunised antibodies that specifically distinguish sarcosine. The subject matter of the invention is also a diagnostic strip for the determination by means of the LFIA method, which is designed for qualitative and quantitative determination of sarcosine in a biological sample. It is convenient for a routine determination of sarcosine in diagnostic laboratories, which may be performed within 2 - 3 hours, utilising equipment commonly available in such laboratories, and it may be also used for self-diagnostics.

## Description

### Technical Field

The invention deals with a qualitative and quantitative determination of sarcosine in a biological sample using anti-sarcosine antibodies, applying nanotechnological labelling with gold nanoparticles possessing peroxidase activity or using quantum dots.

### The State of the Art

Non-protein amino acid sarcosine is involved in amino acid metabolism and methylation processes. A study was performed dealing with urinary sarcosine levels in patients diagnosed with prostate cancer [11,13-15], [8], [10], [16], [17], [18]. In terms of sarcosine detection, enzymatic detection using sarcosine oxidase was described [19]. The usage was described of hydrogen peroxide, which is generated from an enzyme obtained from *Streptomycetaceae* in the sarcosine oxidase reaction at a temperature of 25°C for the enzymatic determination of sarcosine and creatinine [20]. Literature mentions the usage af N-alkyl glycines (their acid amides) and sarcosine anhydride as medical substances suppressing different types of tumours [21]. A unit has been also described for the detection of light intensity emitted from the reactor, related to the sarcosine concentration. The reactor was covered with a layer of a polymer film; a regeneration layer was created between the enzyme catalytic layer and the polymer film layer [22]. Literature also mentions detection of sarcosine by means of electrophoresis, which includes the addition of the buffer solution and pyridium-ruthenium derivative on a capillary device, and the injection of a sample in the capillary applying voltage on capillary ends [23]. In order to determine micro concentrations of sarcosine in a urine sample, a quantitative enzymatic method of detection was applied which uses a peroxidase catalyst and includes a comparison of the fluorescence intensity to the sarcosine concentration [24]. Compositions of lysates were used for the extraction of nucleic acid and detection of microorganisms containing guanidium chloride and N-lauroylsarcosine - sarcosine [25].

At present, an interest has been growing in the implementation of a simple test for the detection of sarcosine level in urine. The above analytical techniques, such as HPLC, microfluid systems, spectrophotometry, (GC/MS) are sensitive, however time-consuming, and they require a rather complex preparation of samples, contrary to the ELISA method (ELISA stands for the *enzyme*-*linkedimmuno sorbent assay*). Such an analytical method is applied for quantitative determination of different antigens on the basis of antigen-antibody interaction. The ELISA method is commonly implemented in clinical-diagnostic laboratories for the determination of a wide spectrum of analytes by means of specific antibodies. Immunoglobulins (IgY) extracted from egg yolks of immunised hens are a suitable alternative of commonly used mammal antibodies extracted from blood. The principal advantages of IgY are as follows: a larger amount of IgY obtained after immunisation; better repeatability of the antibody production for a long-term usage, and a better response of the bird immune system to mammal antigens. Antibodies are often labelled with peroxidase or gold particles possessing peroxidase activity, in which case a spectrophotometric evaluation of the colour reaction is performed after the addition of chromogen; antibodies may be also labelled with quantum dots, in which case fluorescence is evaluated. As it was found out that some groups of chemical substances or nanomaterials, such as gold nanoparticles (AuNPs), might also possess enzymatic (and also peroxidase) activity, these have come to the fore of the research. Gold nanoparticles (AuNPs) have been long used as a tool adequate for molecular-biological experiments. The ELISA method for the quantitative determination of the analyte which uses gold nanoparticles and the evaluation of the colour reaction intensity is mentioned, for example, in the CN105203750 patent. The ELISA sandwich immunoassay for the determination of human protein CA125 using polyclonal antibodies and CdTe quantum dots and fluorescence evaluation is dealt with in the CN103983791 file. Furthermore, as nanotechnologies have been developing fast, nanoparticles are used as one of the principal carriers of biomolecules in nanomedicine and biosensic applications, including applications in experimental cardiology. It is well known that the peroxidase reaction, which takes place in a wide range of biochemical transformations, is widely used in biotechnology. The LFIA test (LateralFlowImmunoAnalysis) has also become of considerable interest; its principle is the combination of chromatography and immunoaffinity reactions. This is a simple tool for the detection of the presence of the analyte in the sample without any necessity to acquire new laboratory equipment. For example, the International patent application WO2015119396 describes an immunochromatographic chip based on this method, which uses catalytic activity of inorganic nanoparticles labelling a specific antibody. The LFIA strip assay using colloid gold particles is, for example, a subject matter of the US2011091906 patent; LFIA for the simultaneous detection of several analytes using the detection by means of quantum dots is described in the WO2006071247 patent application.

However, no fast and easy test has been yet developed for a routine determination of sarcosine using equipment commonly available in diagnostic laboratories, which would be sensitive enough for an early diagnosis of an increased levels of body sarcosine, related, among others, to cancer.

### Literature:

1. Gamagedara, S., et al., Validation study of urinary metabolites as potential biomarkers for prostate cancer detection. Bioanalysis, 2012. 4(10): p. 1175-1183.
2. Goode, R.R., et al., Use of PCA3 in detecting prostate cancer in initial and repeat prostate biopsypatients. Prostate, 2013. 73(1): p. 48-53.
3. Stephan, C., B. Ralla, and K. Jung, Prostate-specific antigen and other serum and urine markers in prostate cancer. Biochimica Et Biophysica Acta-Reviews on Cancer, 2014. 1846(1): p. 99-112.
4. Vickers, A.J. and H. Lilja, We Need a Better Marker for Prostate Cancer. How About Renaming PSA? Urology, 2012. 79(2): p. 254-255.
5. Lucarelli, G., et al., Serum sarcosine increases the accuracy of prostate cancer detection in patients with total serum PSA less than 4.0?ng/ml. Prostate, 2012. 72(15): p. 1611-1621.
6. Jamaspishvili, T., et al., Urine markers in monitoring for prostate cancer. Prostate Cancer and Prostatic Diseases, 2010. 13(1): p. 12-19.
7. Issaq, H.J., T.J. Waybright, and T.D. Veenstra, Cancer biomarker discovery: Opportunities and pitfalls in analytical methods. Electrophoresis, 2011. 32(9): p. 967-975.
8. Gamagedara, S. and Y. Ma, Biomarker analysis far prostate cancer diagnosis using LC-MS and CE-MS. Bioanalysis, 2011. 3(18): p. 2129-2142.
9. Wu, H., et al., GC/MS-based metabolomic approach to validate the role of urinary sarcosine and target biomarkers for human prostate cancer by microwave-assisted derivatization. Analytical and Bioanalytical Chemistry, 2011. 401(2): p. 635-646.
10. Meyer, T.E., et al., A Reproducible and High-Throughput HPLC/MS Method To Separate Sarcosine from alpha- and beta-Alanine and To Quantify Sarcosine in Human Serum and Urine. Analytical Chemistry, 2011. 83(14): p. 5735-5740.
11. Sreekumar, A., et al., Metabolomic profile delineate potential role for sarcosine in prostate cancer progression. Nature, 2009. 457(7231): p. 910-914.
12. Masarik, M., et al., Sarcosine as a new marker for prostate tumours. International Journal of Molecular Medicine, 2010. 26: p. S47-S47.
13. Cernei, N., et al., Sarcosine as a Potential Prostate Cancer Biomarker-A Review. International Journal of Molecular Sciences, 2013. 14(7): p. 13893-13908.
14. Cernei, N., et al., Spectrometric and Electrochemical Analysis of Sarcosine as a Potential Prostate Carcinoma Marker. International Journal of Electrochemical Science, 2012. 7(5): p. 4286-4301.
15. Khan, A.P., et al., The Role of Sarcosine Metabolism in Prostate Cancer Progression. Neoplasia, 2013. 15(5): p. 491-+.
16. Heger, Z., et al., Paramagnetic Nanoparticles as a Platform for FRET-Based Sarcosine Picomolar Detection. Scientific Reports, 2015. 5.
17. Zitka, O., et al., Microfluidic chip coupled with modified paramagnetic particles for sarcosine isolation in urine. Electrophoresis, 2013. 34(18): p. 2639-2647.
18. Zitka, O., et al., Preconcentration based on paramagnetic microparticles for the separation of sarcosine using hydrophilic interaction liquid chromatography coupled with coulometric detection. Journal of Separation Science, 2014. 37(5): p. 465-475.
19. Mayr, U., et al., *Hydrogen peroxide-forming sarcosine oxidase* US Patent-4743549. MAY 10 1988, 1988, Boehringer Mannheim Gmbh. p. 887.
20. Mayr, U., et al., *Method for the determination of sarcosine creatinine or creatinine* US Patent-4845029. JULY 4 1989, 1989, Boehringer Mannheim Gmbh. p. 470.
21. Osswald, H. and M. Youssef, *Use of N low-alkyl glycines their acid amides and of sarcosine anhydride as tumor-inhibiting active substances a remedy containing the former and process for its manufacture* US Patent-4766149. AUGUST 23 1988, 1988, Stiftung Deutsches Krebsforschungszentrum. p. 1942.
22. Ge, Z., H. Yang, and Y. Zhang, Prostate cancer detection device, has inner wall fixed with catalyzing enzyme layer of reactor, and light intensity detecting unit to detect light intensity of light emitted from reactor connected to sarcosine concentration calculation unit, Univ Shenzhen (Uysz-C). p. 5.
23. Li, H. and G. Xu, Detection of sarcosine involves adding buffer solution and pyridinium-ruthenium derivative into capillary electrophoresis device, adding buffer solution and injecting sample into capillary and applying voltage at capillary ends, CHANGCHUN APPLIED CHEM INST CHINESE ACAD (CHAN-Non-standard) CHINESE ACAD SCI CHANGCHUN APPL CHEM INST (CHSC-Non-standard). p. 8.
24. Ge, Z. and H. Yang, Micro sarcosine content quantitative-detection method for e.g. urine sample, involves comparing Sarcosine concentration-fluorescence intensity relationship curves to obtain content of unknown sarcosine sample, Univ Shenzhen (Uysz-C). p. 14.
25. Isac, R., et al., Lysis composition, useful in the extraction of nucleic acid and detection of microorganism, comprises gucanidium chloride and N-lauroyl-sarcosine, MILLIPORE CORP (MIFI-C) ISAC R (ISAC-Individual) MARC F (MARC-Individual) MILLIPORE CORP (MIFI-C) MILLIPORE CORP (MIFI-C) EMD MILLIPORE CORP (MIFI-C). p. 2245157-A1:.

### The Subject Matter of the Invention

The above-mentioned shortfalls are addressed by qualitative and quantitative determination of sarcosine in a biological sample using sarcosine antibodies and gold nanoparticles possessing peroxidase activity, or using sarcosine antibodies and quantum dots, when the detection level of sarcosine is from 0.05 µM to 1.00 µM.

The subject matter of the invention is a method of quantitative determination of sarcosine in a biological sample using anti-sarcosine antibodies and gold nanoparticles, where gold nanoparticles are prepared at 20 ° C; 40 ° C; 60 ° C; 80 ° C or 100° C, their size is from 17 nm to 37 nm, and peroxidase activity is from 0.75 to 0.92 mU/ml. Such nanoparticles are bound to sarcosine antibody or to sarcosine. It is convenient to use gold nanoparticles prepared at 20 ° C with a size from 29 nm to 30 nm. A chromogenous substrate is used for the detection, which contains 0.5 % - 5.0 % of hydrogen peroxide, and an addition of 1-10 mM of auric acid, and 1-20 mM of hydroxylamine hydrochloride, the ratio being 1:1. Quantum dots may be also used for the detection purposes, as these bind to the sarcosine antibody or to sarcosine.

It is convenient to use 3.3',5.5'- tetramethylbenzidine (TMB) as a chromogenous substrate; its concentration should be 0.2 - 1.0 mM, or 4-aminoantipyrine (4AAP) with a concentration 10-50 mM with 3-(N-ethyl-3-methylanilin) propanesulphonic acid sodium salt (TOPS) with a concentration 0.1 - 5.0 mM, or phenylenediamine (OPD) with a concentration 0.5 - 20.0 mM or 2,2'-azino-bis(3-ethylbenzthiazoline-6-sulphonate) (ABTS) with a concentration 1-6 mM, or 5-aminosalicylic acid (5-AS) with a concentration 1-10 mM, or diaminobenzidine (DAB) with a concentration 1-10 mM.

The method of quantitative determination of sarcosine according to the invention (the ELISA assay) may be conveniently implemented in such a manner that a primary anti-sarcosine antibody is fixed on a carrier, a sample containing sarcosine is added, then secondary anti-sarcosine antibody modified with gold nanoparticles is added, which shall bind to sarcosine in the sample; subsequently, auric acid, hydroxylamine hydrochloride and chromogenous substrate are added, and the intensity of the resulting colour is evaluated. The secondary anti-sarcosine antibody may be also modified with quantum dots; in such a case, the intensity of fluorescence is evaluated after the final incubation of all the components during 1 hour at 37°C in the dark in order to increase the reaction response.

In compliance with this invention, quantitative determination of sarcosine may be also conveniently performed by means of fixing primary anti-sarcosine antibody on a carrier, adding a sample containing sarcosine, and subsequently adding sarcosine modified with gold nanoparticles, and after that, chromogenous substrate, auric acid and hydroxilamine hydrochloride are added and the intensity of resulting colour is evaluated. Sarkosine may be also modified with quantum dots; in such a case, after the final incubation of all the components during one hour at 37°C in the dark, in order to increase the reaction response, intensity of fluorescence is evaluated.

In compliance with the invention, quantitative determination of sarcosine may be also performed in an accelerated manner when the evaluation is done in two hours; in such a case, secondary antibody of the primary anti-sarcosine antibody is fixed to the carrier, then a sample containing sarcosine is added, then primary anti-sarcosine antibody is added, then a anti-sarcosine antibody modified with gold nanoparticles is added, and after that, chromogenous substrate, auric acid and hydroxilamine hydrochloride are added and the intensity of resulting colour is evaluated. Instead of being modified with gold nanoparticles, anti-sarkosine antibody may be also modified with quantum dots; in such a case, after the final incubation of all the components during one hour at 37°C in the dark, in order to increase the reaction response, intensity of fluorescence is evaluated. As a carrier, a microtitration plate, magnetic particles or another adequate carrier may be conveniently used.

A subject matter of the invention is also a diagnostic strip for the determination of sarcosine in a biological sample applying the LFIA (LateralFlowImmunoAnalysis) method; for such a purpose, anti-sarcosine antibody labelled with peroxidase-active gold nanoparticles or quantum dots are used. For urine testing, first it is necessary to determine the quantity of creatinine in the sample, the concentration of which should be from 5 mM to 10 mM, which is a dilution level suitable for a reliable determination of sarcosine with the diagnostic strip. If the dilution is higher, the determination of sarcosine cannot be performed. The diagnostic strip is composed of a rigid pad containing a sample zone at one end, composed of one sample zone of glass fibres on which a zone of glass fibres is applied containing primary anti-sarcosine antibody labelled with gold nanoparticles or quantum dots, and of the second zone of glass fibres applied on the glass fibre zone containing the labelled primary antibody; the zone with a labelled antibody lengthwise overlaps both layers of glass fibres. Behind the zone containing the labelled antibody, a membrane is located towards the other strip end on which a control zone containing an antibody of the primary anti-sarcosine antibody is applied transversally towards the strip, and behind it, there is a testing zone containing secondary anti-sarcosine antibody. Behind the membrane, an absorption zone follows which forms the other end of the strip.

Another subject matter of the invention is a manner of determination of sarcosine in a biological sample using the above-mentioned diagnostic strip, where a sarcosine detection limit is from 0.05 µM to 1.00 µM. If the strip contains primary anti-sarcosine antibody labelled with gold nanoparticles, the strip is dipped into a developer containing NaCl with a concentration 0.09-0.20 M, KCl with a concentration 2-5 mM, Na₂HPO₄ with a concentration 5 - 10 mM, KH₂PO₄ with a concentration 1-3 mM, bovine serum albumin with a concentration 0.2-0.8 %, polyoxyethylene(20)-sorbitan-monolaurate (Tween20) with a concentration 0.5-2.0%, the sample, addition of 1-10 mM of auric acid, and 1-20 mM of hydroxylamine hydrochloride with a ratio of 1:1, and when 15 minutes pass, the colour intensity within the testing zone is evaluated.

Primary anti-sarcosine antibody may be also labelled with quantum dots in the strip. In such a case, the strip is dipped in a developer containing NaCl with a concentration 0.09-0.20M, KCl with a concentration 2-5 mM, Na₂HPO₄ with a concentration 5 *-* 10 mM, KH₂PO₄ with a concentration 1-3 mM, bovine serum albumin with a concentration 0.2-0.8 %, polyoxyethylene(20)-sorbitan-monolaurate (Tween20) with a concentration 0.5-2%, the sample, addition of 1-10 mM of auric acid, and 1-20 mM of hydroxylamine hydrochloride with a ratio of 1:1, and when 15 minutes pass, the fluorescence intensity within the testing zone is evaluated.

The diagnostic strip which is subject matter of the invention has a convenient length of 6.0 cm and width of 0.5 cm, and the sample zone, the first and second glass fibre zones are 2.0 cm long. After the sample zone, a 0.3 cm long zone containing labelled antibody follows, followed by a 2.2 cm long membrane to which a control zone is applied, after which, at a distance of 5.0 mm, a testing zone is applied, and after the membrane, there is absorbent zone 1.5 cm long, which forms the other end of the strip.

The diagnostic strip may also have a plastic pad with a nitrocellulose membrane, on which control and testing zones are applied. The testing zone contains anti-sarcosine antibodies, however it contains different parts of the molecule than the primary antibody labelled with gold nanoparticles, which is a part of the conjugate located immediately after the start of the test and which migrates through the strip. The control zone contains antibodies of the primary anti-sarcosine antibody. Testing and control lines are conveniently applied to the nitrocellulose membrane using a printer. The conjugate of glass fibres with the fixed anti-sarcosine antibody and all the other zones are glued on a plastic pad.

The biological sample may be, for example, urine, serum, plasma or sperm and other body liquids, cell lysates, etc. In case of a quantitative determination of sarcosine in urine with the diagnostic strip, the creatinine level in the urine sample should be from 5 mM to 10 mM.

### List of Figures

Fig. 1: Visible colour changes of studied nanoparticles with 0.2-1mM 3.3',5.5'-tetramethylbenzidine (TMB) and H₂O₂, From the left, the figure always shows a vial of TMB H₂O₂ without nanoparticles, gold nanoparticles (AuNPs) prepared at a certain temperature, and the last vial always shows the colour reaction with particles. (A) AuNPs 20°C; (B) AuNPs 40°C; (C) AuNPs 60°C; (D) AuNPs 80°C; (E) AuNPs 100°C; (F) control peroxidase activity I mM HRP.
Fig. 2: Description of the Method of Binding of Antibodies and Sarcosine.
   (A) 1. Plate coated with Anti-Sar antibody. 2. Surface blocking with commonly used reagents (BSA). 3. Antigen (sarcosine) bound to antibodies. 4. AuNPs conjugate with Anti-Sar antibody bound to antigen (sarcosine). 5. The addition of auric acid and hydroxilamine in order to strengthen the detected signal. 6. Substrate with TMB and H₂O₂ turn blue in the presence of AuNPs.
   (B) 1. Plate coated with Anti-Sar antibody. 2. Surface blocking with commonly used reagents (BSA). 3. Antigen (sarcosine) bound to antibodies. 4. AuNPs conjugate with sarcosine bound to free antibodies on the plate. 5. Addition of auric acid and hydroxilamine. 6. Substrate with TMB and H₂O₂ creates colouring in the presence of AuNPs; in such a case, the result of sarcosine detection in the sample is negative. 7. Antibody binding sites on the plate were occupied by sarcosine contained in the sample without AuNPs bound to it, there is no colouring and the result of sarcosine detection in the sample is positive.

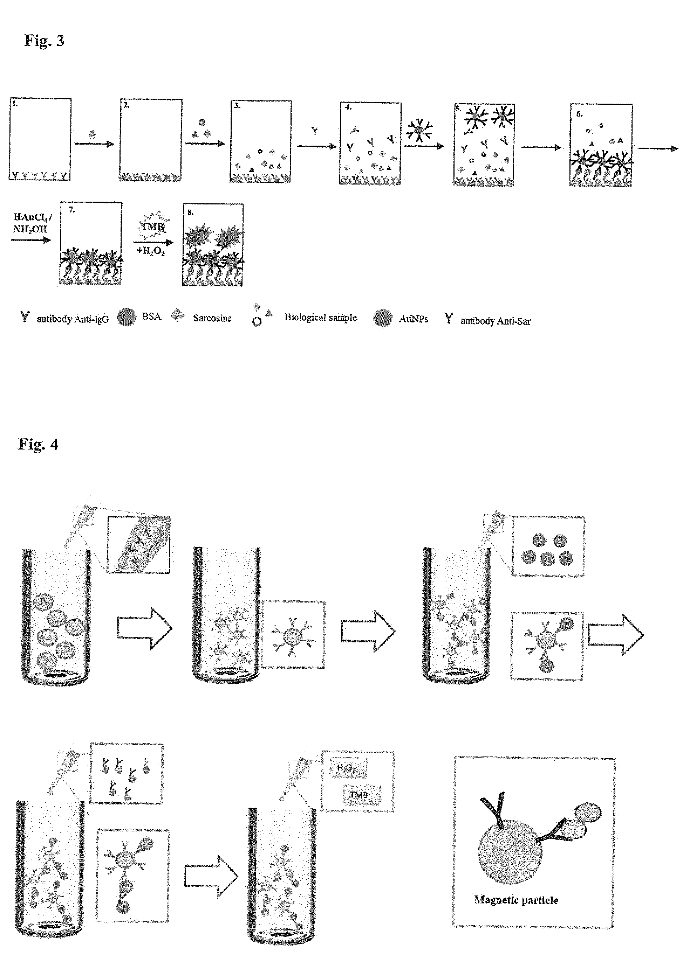

Fig. 3: Description of an accelerated binding of antibodies and sarcosine. 1. The plate is coated with an antibody against the primary antibody. 2. Blocking of the reaction surface (albumin, milk protein, egg white protein, etc.). 3. Addition of a sample containing the analyte (sarcosine). 4. Primary antibody of the sample. 5. Secondary labelled antibody. 6. Creation of a complex of antibodies with target molecules and its strong bond. 7. Addition of auric acid and hydroxilamine in order to strengthen the detection system. 8. Detection step, peroxidase activity, quantum dot, etc.
Fig. 4: Description of the binding of antibodies and sarcosine if magnetic particles are used as the carrier
   1. Magnetic particles with a surface treated for the binding of antibody.
   2. Magnetic particles are modified with the AntiSar primary antibody.
   3. Binding of the analyte (sarkosine) to the magnetic particle.
   4. Application of the secondary antibody with bound gold nanoparticle/quantum dot.
   5. Application of substrate for the measuring of peroxidase activity/fluorescence.
   6. Direct detection of analyte labelled with gold nanoparticle due to its bond to the primary antibody.

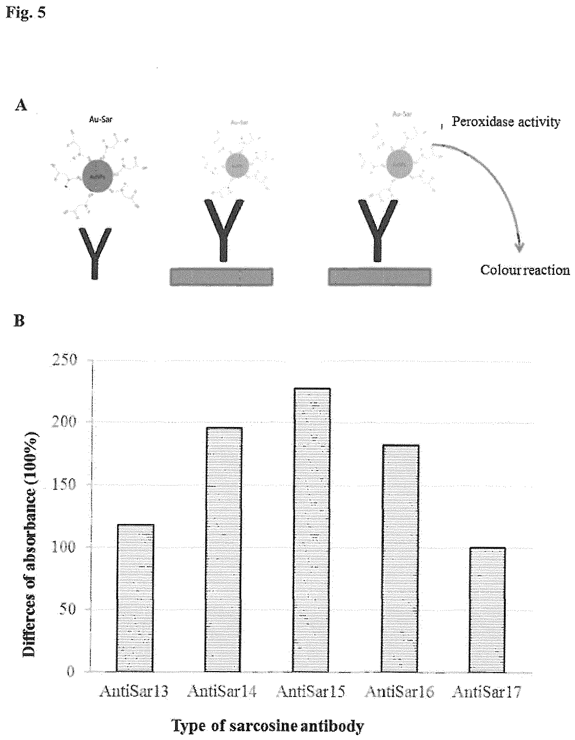

Fig. 5: (A) Sarcosine-modified gold nanoparticles and their bond to the AntiSar antibody fixed on an adequate carrier; after the binding of antibodies on sarcosine, peroxidase activity of gold nanoparticles is utilised and it is transferred to suitable colour substrates.
   (B) Affinity of different types of AntiSar antibodies in the Au-sarkosine bond (100 µM). AntiSar 17 activity has been chosen to be 100%; 0.1 AU.

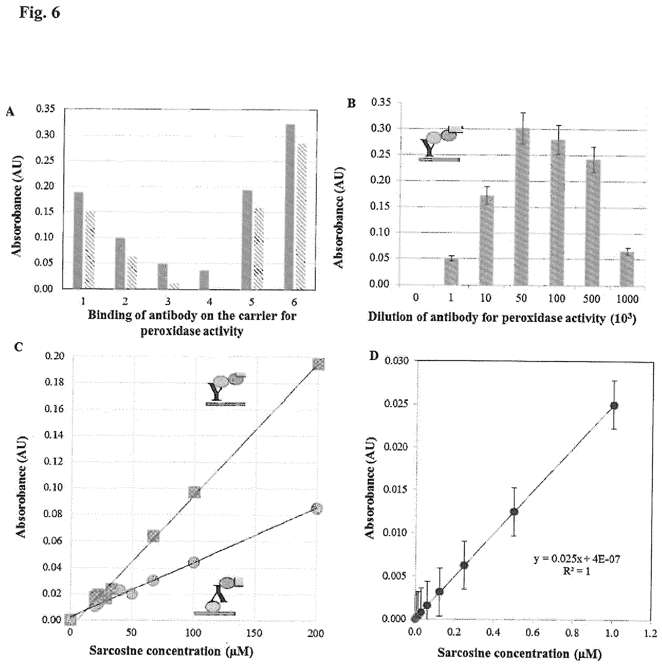

Fig. 6: Dependence of peroxidase activity of gold nanoparticles utilising suitable substrates (TMB) on the changing concentration of sarcosine.
   (A) Non-labelled sarcosine is bound on the plate, and labelled Au-Anti-Sar 15 (y = 0,001x - 0,0055, R² = 0,9959) antibody is used for the detection; (1) Sarcosine is bound on the plate and an antibody with a gold particle is bound to it (2); Empty well in which auric acid was added (3); Only substrate is present in an empty well (4); Antibody labelled with gold (5); Bound golden sarcosine (6).
      Monitoring of the antibody bond with a peroxidase-active carrier. The first column shows the general peroxidase activity obtained; the second column shows the reading of the used substrates. The activity was monitored as changes of the TMB signal.
   (B) Unlabelled Anti-Sar 15 antibody is bound to the plate, and gold sarcosine is used for the detection; (y = 0,0004x + 0,0021, R² = 0,9944). Dilution of the antibody for the purposes of monitoring of peroxidase activity of gold nanoparticles bound to a solid carrier. Quantity of antibody was changed; sarcosine concentration (10 µM). The activity was monitored as changes of the TMB signal.
   (C) Comparison of calibration curves of sarcosine detection applying the competitive ELISA method using labelled sarcosine and the sandwich ELISA and labelled antibody.
   (D) Dependence of peroxidase activity absorbance on a low sarcosine concentration. Theactivity was monitored in a form of changes of the TMB signal. Sarcosine detection limit was 0.05 µM.

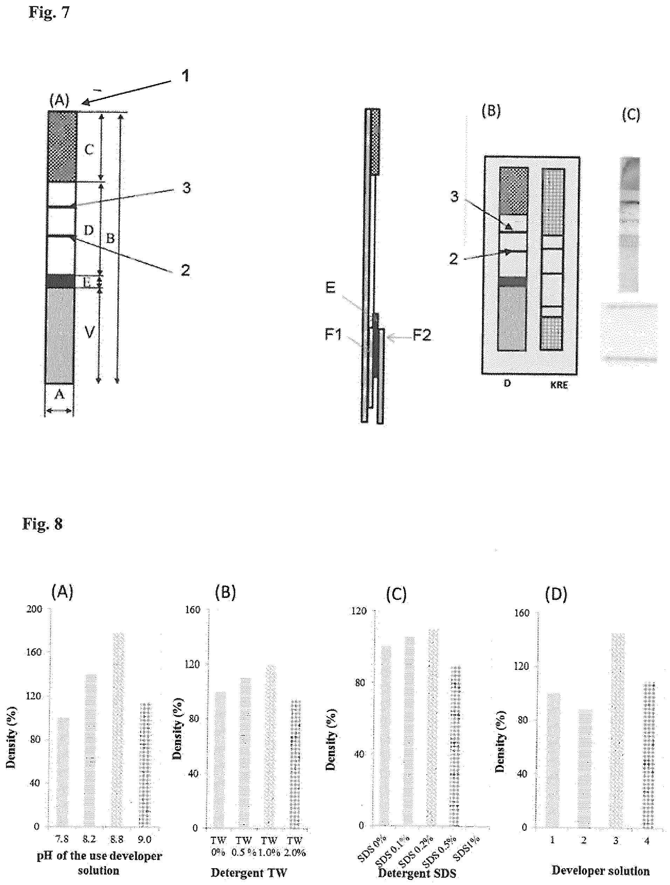

Fig. 7: Structure of the Strip Detection Test.
   (A) Left - detection strip with the arrangement of the individual zones B- length 6.0 cm; A- width 0.5 cm; V - sample zone, length 2.0 cm; E - zone composed of glass fibres containing antibodies with gold nanoparticles, length 0.3 cm; D - membrane containing a control zone (2) and a testing zone (3), length 2.2 cm; C - absorption zone, length 1.5 cm; Right - detection strip - side view; layout of the sample zone layers V. A filter of glass fibres is located on an adhesive plate - first zone F1 - then filter of glass fibre (conjugation) - zone E with labelled antibody (Au-AntiSar; QD-AntiSar), and overlapped with a filter of glass fibres - the second zone F2. (B). The testing system for urine is arranged in a form of D KRE, where KRE is a paper detection strip for creatinine, D is a strip test for sarcosine with two lines: positive control, testing zone; (C) result of the strip detection test which utilises gold nanoparticles aggregation, peroxidase activity of these, or fluorescence of quantum dots. Upper line of the testing zone; the detail view below shows the testing zone: up - before the colour reaction, down - after the reaction (substrates are applied in even layers on the surface).
Fig. 8: Changes in the intensity of the aggregation of gold nanoparticles in a detection strip at the site of the AntiSar antibodies bond (A) The influence of pH of the used borate buffer; (B) Influence of the used TW detergent; (C) Influence of the used SDS detergent; Developer solution in order to maximise the response. Concentration of sarcosine 10 µM; concentration of Antisar antibody 1 µg. Mean determination error 12%.

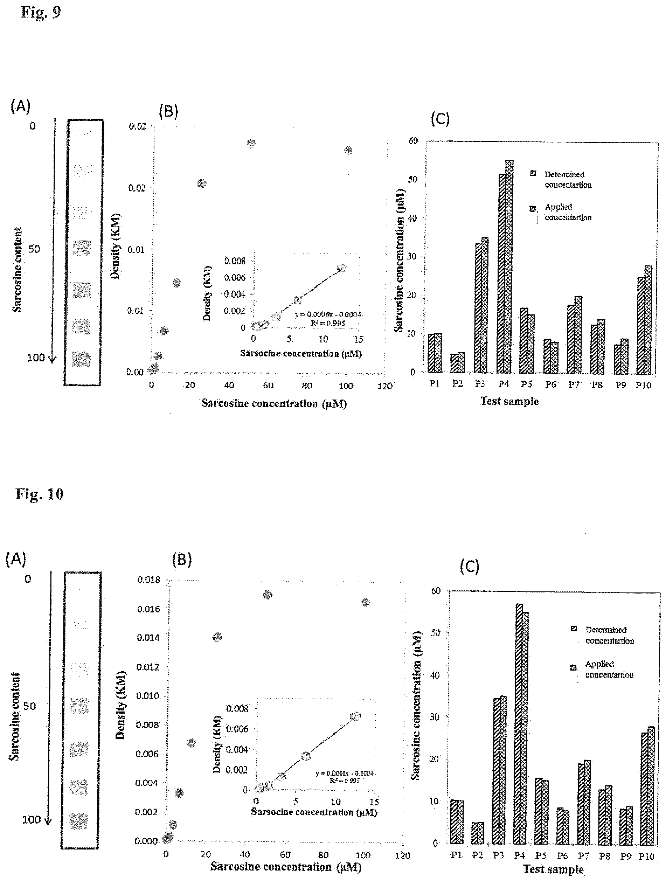

Fig. 9: Detection strip for the determination of sarcosine quantity in a buffered environment. (A) Detection colour scale used for the evaluation of the quantity of sarcosine, coloured due to the aggregation of gold nanoparticles / quantum dots; (B) Dependence of sarcosine quantity on the colour reaction determined density (KM); in the inset - linear part of the dependence (R2 = 0,995); (C) Known quantities of sarcosine in test samples in buffered environment - hatched graph shows the comparison of the determination of sarcosine concentration in such samples with a detection strip, as applied concentrations.
Fig. 10: Detection strip for the determination of a sarcosine quantity in artificial urine (sodium chloride with a concentration 100-200 mM; potassium chloride with a concentration 30-80 mM; sodium phosphate with a concentration 10-50 mM; urea with a concentration 200-500 mM, creatinine with a concentration 10-25 mM, bovine serum albumin with a concentration 600-800 µM); (A) Detection colour scale used for the evaluation of the quantity of sarcosine, coloured due to the aggregation of gold nanoparticles / quantum dots; (B) Dependence of sarcosine quantity on the colour reaction determined density (KM), in the inset - linear part of the dependence (R² = 0,995); (C) Known quantities of sarcosine in test samples of artificial urine - hatched graph shows a comparison of the determination of the sarcosine concentration in such samples with a detection strip, as applied concentrations. Mean determination error 15%.

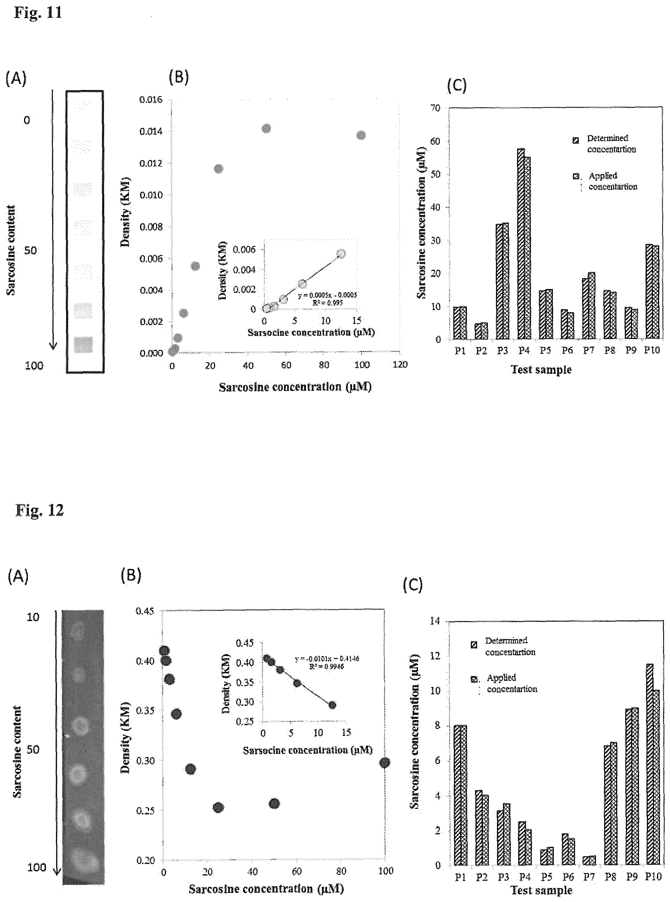

Fig. 11: Detection strip for the determination of a sarcosine quantity in urine. (A) Detection colour scale used for the evaluation of the quantity of sarcosine, coloured due to the aggregation of gold nanoparticles; (B) Dependence of sarcosine quantity on the colour reaction determined density (KM), in the inset - linear part of the dependence (R² = 0,995); (C) Known quantities of sarcosine in test samples of urine - hatched graph shows the comparison of the determination of the sarcosine concentration in such samples with a detection strip, as applied concentrations. Mean determination error 18%.
Fig. 12: Detection strip for the determination of a sarcosine quantity in urine using labelling with quantum dots. (A) Detection colour scale used for the evaluation of the quantity of sarcosine, coloured due to the fluorescence of quantum dots; (B) Dependence of sarcosine quantity on the colour reaction determined density (KM), in the inset - linear part of the dependence (R² = 0,9945); (C) Application of a detection strip for the determination of sarcosine in the test sample of artificial urine - hatched graph shows the comparison of the determined and applied sarcosine concentrations. Mean determination error 10.5 %.

The invention is described in further details giving examples of its implementation, which in no manner limit any possibilities covered by the patent claims.

### Examples of Making the Invention

### Preparation of Gold Nanoparticles; Labelling of Antibodies with Peroxidase-Active Gold Nanoparticles

Gold nanoparticles were prepared from 0.5-3.0mM solution of HAuCl₄. 3H₂O and a solution of trisodium citrate with a concentration 0.05-0.2 M under constant stirring during 1 hour at 20°C on a magnetic blender (100 rpm) in a beaker covered with a watch-glass.

The formation of gold nanoparticles was evidenced by an observable change of the yellow colouring into violet. Subsequently, gold nanoparticles were prepared by means of a thermal synthesis at 40°C, 60°C, 80°C and 100 °C applying the same steps as at 20 °C. Particles prepared at 20°C are the most easy to prepare and the most suitable for the method which is the subject-matter of the invention; other suitable alternatives which can be used are particles thermally prepared at temperatures 40 °C, 60 °C, 80 °C and 100 °C.

Then, particles bound to antibodies. Borate buffer with pH 8.8 (3-6 mM) was transferred with a pipette into a beaker, and under constant stirring, gold nanoparticles were added (AuNPs). Then, small dosages of antibodies were added with a concentration of 1mg/ml. Afterwards, the solution was incubated during 90 minutes at 37°C under constant stirring. When the incubation period finished, the solution was centrifuged at 10°C during 15 minutes at 13600 x g. Supernatant was removed with a pipette, and flushing solution was added to the pellets and centrifugation was again performed. Such a procedure was repeated with 0.5 ml of flushing solution and centrifugation was performed under the same conditions. Then, storage solution was added to the pellet. A conjugate prepared in such a manner was stored at 4 °C in the dark.

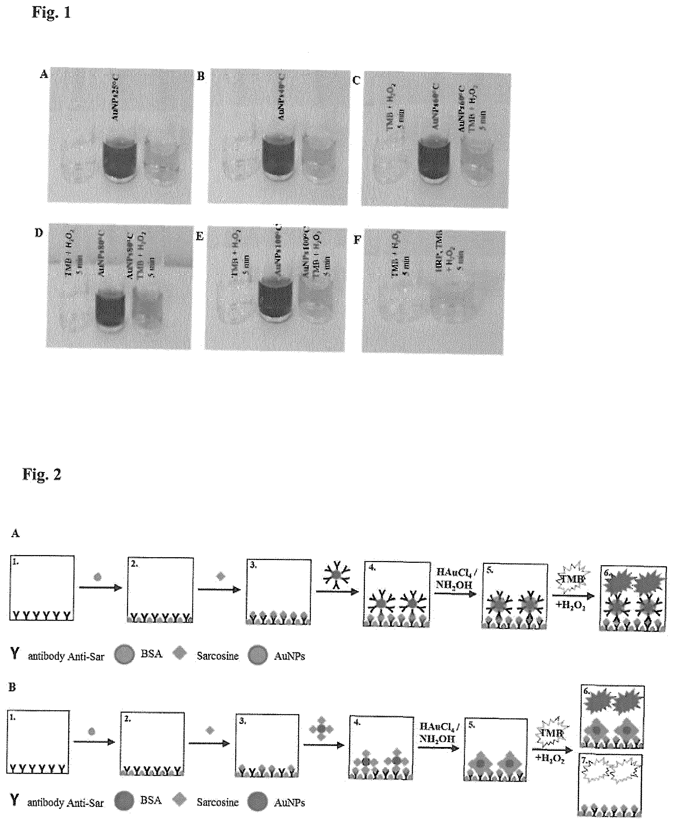

Particles obtained were characterised by means of available physical-chemical procedures. Results obtained are summarised in Tab. 1. The evaluation of the AuNPs peroxidase activity was normalised to the activity of horseradish peroxidase (HRP) and substrate. In all the AuNPs types, the peroxidase activity was from 0.75 to 0.92 mU/ml (Fig.1).

**Tab. 1 Basic characterisation of thermally prepared gold nanoparticles.**

| **AuNPs prepared at** | **AuNPs 20°C** | **AuNPs 40°C** | **AuNPs 60°C** | **AuNPs 80°C** | **AuNPs 100°C** |
|---|---|---|---|---|---|
| pH | 5.1 | 4.9 | 5.0 | 5.0 | 5.0 |
| Particle size [nm] | 29 | 29 | 37 | 17 | 17 |
| Zeta potential [mV] | -37.9 | -32.0 | -32.4 | -43.9 | -37.3 |
| Absorption maximum [nm] | 531 | 529 | 529 | 529 | 529 |

In a biological experiment, effect of nanoparticles on H9C2 cell culture was assessed. AuNPs were applied to cell culture during the exponential phase of growth. No toxicity effects on the cell culture were observed.

### Implementation of the ELISA Test

A microtitration plate was coated with the primary anti-sarcosine specific antibody in a quantity from tens to hundreds nanograms per one microtitration-plate well. The binding of the antibody was performed in 0.01 - 1.00 M carbonate buffer with pH 9 at 37°C during 1 hour. Afterwards, the liquid was evacuated and the plate surface was blocked with 1 -5 % solution of albumin or dried egg white or dried milk during 40 minutes at 37°C. A plate blocked in such a manner was flushed with PBS-T buffer, a biological sample containing sarcosine was added with a pipette (urine, serum, plasma and other body liquids, cell lysates, etc.), and incubation was performed during 1 hour at 37°C. After incubation, the sample was sucked off from the plate and flushed again with the PBS-T buffer. Then, the secondary antibody labelled with gold nanoparticles and diluted in a ratio 1:20 - 10,000 was added with a pipette, and incubation was performed during 1 hour at 37°C. Then, the plate was flushed with distilled water. In order to increase the peroxidase activity of gold nanoparticles, 1-10 mM of auric acid and 1-20 mM of hydroxylamine hydrochloride was added in a ratio 1:1, which was left during 20 minutes to react at room temperature. The principal implementation of the method of sarcosine detection applying the ELISA method are summarised in Fig. 2. After 20 minutes of incubation at a room temperature and flushing with distilled water, a chromogenous substrate was added. Colour reaction took place, and using a spectrophotometer, absorbance of samples on the plate was measured.

### Example 1

### Determination of Sarcosine in a Urine Sample Applying the ELISA Test and a 3.3',5.5'- tetramethylbenzidine Detection System

The determination was performed applying the above-mentioned procedure. After 20 minutes of incubation at room temperature, the plate was flushed with distilled water, and a substrate was added with a pipette containing 0.2 - 1.0 mM 3.3',5.5'- tetramethylbenzidine (TMB) and 0.5 - 5.0% hydrogen peroxide. Colour reaction took place, and using a spectrophotometer, absorbance of samples on the plate was measured at 655 nm.

### Example 2

### Determination of Sarcosine in a Serum Sample Applying the ELISA Test and a 4-minoantipyrine Detection System

The ELISA test was performed applying the above-mentioned procedure. When the process finished, the plate was flushed with distilled water, and a substrate was added with a pipette containing 10-50 mM4- of aminoantipyrine (4AAP) with 0.1 - 5 mM sodium salt 3-(N-ethyl-3-methylaniline) of propane sulphonic acid (TOPS) and 0.5 - 5.0% hydrogen peroxide. Colour reaction took place, and using a spectrophotometer, absorbance of samples on the plate was measured at 535 nm.

### Example 3

### Determination of Sarcosine in a Plasma Sample Applying the ELISA Test and a O-phenylenediamine Detection System

The ELISA test was performed applying the above-mentioned procedure. When the process finished, the plate was flushed with distilled water, and a substrate was added with a pipette containing 0.5 - 20 mM of O-phenylenediamine (OPD) and 0.5 - 5.0% hydrogen peroxide. Colour reaction took place, and using a spectrophotometer, absorbance of samples on the plate was measured at 450 nm.

### Example 4

### Determination of Sarcosine in a Sperm Sample Applying the ELISA Test and a 2,2'-azino-bis(3-ethylbenzthiazoline-6-sulphonate) Detection System

The ELISA test was performed applying the above-mentioned procedure. When the process finished, the plate was flushed with distilled water, and a substrate was added with a pipette containing 1-6 mM 2,2'-azino-bis (3-ethylbenzothiazoline-6-sulphonic acid) (ABTS) and 0.5 - 5.0% hydrogen peroxide. Colour reaction took place, and using a spectrophotometer, absorbance of samples on the plate was measured at 425 nm.

### Example 5

### Determination of Sarcosine in a Urine Sample Applying the ELlSA Test and a 5-Aminosalycilic Acid Detection System

The ELISA test was performed applying the above-mentioned procedure. When the process finished, the plate was flushed with distilled water, and a substrate was added with a pipette containing 1-10 mM of 5-aminosalicylic acid (5-AS) and 0.5 - 5.0% hydrogen peroxide. Colour reaction took place, and using a spectrophotometer, absorbance of samples on the plate was measured at 530 nm.

### Example 6

### Determination of Sarcosine in a Serum Sample Applying the ELISA Test and a Diaminobenzidine Detection System

The ELISA test was performed applying the above-mentioned procedure. When the process finished, the plate was flushed with distilled water, and a substrate was added with a pipette containing 1-10 mM diaminobenzidine (DAB) and 0.5 - 5.0% hydrogen peroxide. Colour reaction took place, and using a spectrophotometer, absorbance of samples on the plate was measured at 670 nm.

### Example 7

### Shortened ELISA Method Lasting 90 Minutes, Detection System as per Examples 1-6

The microtitration plate was coated with an antibody specific against the primary antibody. Binding was performed in 0.01 - 1.00 M of carbonate buffer with pH 9 at 37°C during 1 hour. Afterwards, the liquid was sucked and the plate surface was blocked with 1 -5 % solution of albumin or dried egg white and dried milk during 40 minutes at 37°C. A plate blocked in such a manner was flushed with PBS-T buffer; biological sample containing sarcosine was added with a pipette (urine, plasma, serum and sperm), together with the sarcosine-specific primary antibody and sarcosine-specific secondary antibody and labelled with gold nanoparticles. Such a blend was incubated on the plate during 1 hour at 37°C.

Afterwards, the plate was flushed with distilled water, and 1-10 mM of auric acid and 1-20 mM of hydroxylamine hydrochloride was added in a ratio 1:1; such an addition was left during 20 minutes to bind at room temperature (Fig. 3). Flushing with distilled water was again performed and substrate was added with a pipette, and measurement was performed as per examples 1-6.

### Example 8

### Implementation of the Method in According to the Intention, Using Antibodies Modified with Gold Nanoparticles on Magnetised Nanoparticles

Magnetic particles were prepared which were subsequently modified with a sarcosine-binding antibody. Secondary antibody labelled with gold nanoparticles or quantum dots was added to such a mixture (Fig.4). Peroxidase activity of the sample was monitored using substrates and applying the above-mentioned evaluation method.

The preparation of magnetic nanoparticles was performed as follows: in a beaker, 1-3 g Fe(NO₃)₃.9 H₂O was dissolved using distilled water. Then, 0.3-0.7M NaBH₄ dissolved in 3.5 % NH₃ was gradually added with a pipette. The beaker was covered with a watch glass and the solution was heated during 2 hours at 100 °C being continuously stirred on a magnetic blender. After the lapse of the established time, the solution was stirred at room temperature during additional 24 hours. The resulting product, which was fixed with a magnet on the bottom of the beaker, was flushed 3 times with distilled water. Then, a solution of HAuCl₄ . 3H₂O with a concentration 0.5-3.0 mM was added in the beaker; the solution was stirred for 3 hours, and then, trisodium citrate with a concentration 0,05-0,2 M was added. Stirring was performed during 24 hours. The product was fixed with a magnet and was flushed 3 times with distilled water. After pouring out water and non-fixed nanoparticles, fixed nanoparticles were dried in a magnetic blender at 40 °C (1 hour). Then particles were scraped off and ground.

CdTe quantum dots were prepared stirring a solution of Cd(CH₃COO)₂ · 2H₂O with a concentration 0.01-0.05 M, distilled water, mercaptosuccinic acid (MSA) solution with a concentration 0.2-0.6 M, NH₃ solution with a concentration 0.5-2.0 M, Na₂TeO₃ solution with a concentration 0.01-0.04 M and 30-50 mg of NaBH₄ on a magnetic blender. Stirring was performed at least during 2 hours, until bubbles stopped forming. Then, the volume was adjusted to 100 ml. Prepared solution was transferred with a pipette into vials which were closed with white caps and then Teflon caps were screwed on. Vials prepared in such a manner were put in a microwave oven set to 300 W and heating was performed for 3 minutes. The resulting colour of CdTe particles was green.

Prepared CdTe quantum dots were used for conjugation with the antibody in such a manner that first, CdTe were conjugated in 2-propanol in 1:1 ratio. Centrifugation followed at 14,000 xg 5 min at laboratory temperature. After that, supernatant was removed with a pipette, and distilled water was added to the pellet, to which a solution of N-(3-dimethylaminopropyl)-N' -ethylcarbodiimide hydrochloride (EDC) with a concentration 1.5-3.5 mM and a solution of N-hydroxysuccinimide (NHS) with a concentration 3-6 mM and methanol were added. Incubation at laboratory temperature during 30 minutes was performed. A solution of 10 times diluted antibodies was added to such a solution. Incubation followed at laboratory temperature in the dark during a minimum period of 4 hours. The resulting product was stored at 4 °C.

### Example 9

### Preparation of the Diagnostic Strip

Diagnostic strip 1 was prepared from an adhesive pad with an included nitrocellulose membrane (Whatman, GE Healthcare Life Sciences, UK), of length B 6.0 cm and width A 0.5 cm. The sample zone V was 2.0 cm long and it was composed of three layers (on the adhesive pad, a filter of glass fibres was placed - first zone F1 2 cm long, then a filter of glass fibres (conjugation filter) with the labelled antibody (Au-AntiSar; QD-AntiSar) - zone E, which was overlapped with a 2 cm long glass fibre filter - the second F2 zone. Zone E overlapped the first F1 zone and also the second F2 zone by 0.3 cm. The prepared layers of sample zone V may be dried at laboratory temperature or a slightly raised temperature, applying low pressure and using lyophilisation.

The complete sample zone was covered with an inert strip so that liquid could enter the detection strip only laterally. On nitrocellulose membrane D 2.2 cm long, which followed zone E, antibodies were applied in control zone 2- antibody against antibody, and at a distance of 5 mm from control zone 2, AntiSar antibody was applied in testing zone 3. Immediately after membrane D, in absorption zone C, filtration paper Whatman 1 (Whatman, GE Healthcare Life Sciences, United Kingdom) 0.5 cm wide and 1.5 cm long was placed. Considering a long-term usage, it is recommendable to store the strip 1 in a dry, dark and cold place with a reduced oxygen concentration. For the purposes of the assessment of the suitability of the proposed test performed with such a detection strip, the detection test has been amplified with a zone for the creatinine level monitoring (Fig.7B, on the right). Especially when testing urine, it is first necessary to determine the level of creatinine in the sample, which should be in a range from 5 mM to 10 mM, which is an evidence of an adequate dilution of the sample. If dilution is higher, such a detection of sarcosine cannot be preformed.

The change in the colour in testing zone 3 occurs due to the aggregation of peroxidase-active gold nanoparticles or quantum dots with fluorescent properties.

### Determination of Sarcosine in Buffered Environment and in a Urine Sample

For testing purposes, a sample of fresh, in the best case, first-morning, urine should be used, and such a sample should be stirred. First, level of creatinine was tested in the urine sample in order to verify the adequate dilution of the sample for the purposes of the determination of sarcosine. The level of creatinine was tested using a diagnostic strip (Fig. 7B), composed, starting from the end, of the sample zone, two reaction zones, detection zone, and a zone of beeswax on the other end, on the basis of a test that uses creatininase enzyme, creatinase and sarcosine oxidase with formation of hydrogen peroxide, which, due to the peroxidase reaction and chromogenous substrate, creates colouring proportional to the level of creatinine in the sample. The sample zone of the creatinine diagnostic strip was dipped in the sample and was left during 15 minutes to absorb the sample, and then, the intensity of the colouring was evaluated. The determined creatinine level in the sample was in the required range from 5 mM to 10 mM.

Subsequently, determination of sarcosine in the sample was performed using a diagnostic strip prepared as mentioned above. Strip 1 containing antibody labelled with gold nanoparticles with sample zone V was dipped in a developer solution with an addition of the sample; the developer solution contained NaCl with a concentration 0.09-0.20M, KCl with a concentration 2-5 mM, Na₂HPO₄ with a concentration 5 - 10 mM, KH₂PO₄ with a concentration 1-3 mM, bovine serum albumin with a concentration 0.2-0.8 %, polyoxyethylene(20)-sorbitan-monolaurate (Tween20) with a concentration 0.5-2%, the sample, addition of 1-10 mM of auric acid, and 1-20 mM of hydroxylamine hydrochloride in a ratio 1:1.

In 15 minutes, the result was evaluated by means of the measuring of the intensity of colouring of the testing zone 3. The colour of testing zone 3 was scanned and then evaluated using the Qinslab application (colour test) (Fig. 9, 11).

### Example 10

### Detection Strip System for the Determination of Sarcosine in Different Environments

A detection strip composed as described above was used for testing. Its characteristics for the determination of sarcosine in different environments were evaluated - in buffered environment, artificial urine and real urine sample (Fig. 9-11). In order to further enhance the detection properties of gold nanoparticles aggregation, it is convenient to use an addition of auric acid (1-10 mM) and hydroxylamine hydrochloride (1-20 mM) in developer solution, which contains NaCl with a concentration 0.09-0.20 M, KCl with a concentration 2-5 mM, Na₂HPO₄ with a concentration 5 - 10 mM, KH₂PO₄ with a concentration 1-3 mM, bovine serum albumin with a concentration 0.2-0.8 %, and polyoxyethylene(20)-sorbitan-monolaurate (Tween20) with a concentration 0.5-2%.

### Industrial Applicability

The test is suitable for a routine determination of sarcosine in a biological sample, especially urine, in diagnostic laboratories, and also for self-diagnostics. This test can provide information of the level of sarcosine in an unknown sample with a high sensitivity. The determination can be preformed within 2 - 3 hours while using equipment commonly available in such laboratories. As compared to common determination methods (analysis of amino acids by means of liquid chromatography), the determination time is importantly reduced, and only a minimum treatment of the sample is required.

### List of denomination marks

1 - diagnostic strip
2 - control zone
3 - testing zone
V - sample zone
F1 - first glass fibre zone
F2 - second glass fibre zone
E - glass fibre zone containing labelled antibody
D - membrane
C - absorption zone

## Claims

1. The method of quantitative determination of sarcosine in a biological sample by means of anti-sarcosine antibodies and ;-active gold nanoparticles, or by means of anti-sarcosine antibodies and quantum dots, **which is characterised by the fact that** the sarcosine detection limit is from 0.05 µM to 1.00 µM.

2. The method of quantitative determination of sarcosine by means of anti-sarcosine antibodies and peroxidase-active gold nanoparticles as per Claim 1, **which is characterised by the fact that** gold nanoparticles are prepared at 20 ° C, 40 ° C, 60 ° C, 80 ° C or 100 ° C, with a size from 17 nm to 37 nm, with peroxidase activity from 0.75 mU/ml to 0.92 mU/ml, where the detection of the sarcosine level is performed using chromogenous substrate which contains 0.5 % - 5.0 % of hydrogen peroxide and an addition of 1 mM -10 mM of auric acid and 1 mM-20 mM of hydroxylamine hydrochloride in a ratio 1:1.

3. The method of quantitative determination of sarcosine as per Claim 2, **which is characterised by the fact that** the chromogenous substrate is 3,3',5,5'-tetramethylbenzidine with a concentration from 0.2 mM - 1.0 mM or 4- minoantipyrine with a concentration 10 mM -50 mM with a sodium salt 3-(N-ethyl-3-methylaniline) of propanesulphonic acid with a concentration 0.1 mM - 5.0 mM, or O-fenylendiamine with a concentration 0.5 mM - 20.0 mM, or 2,2'-azino-bis(3-ethylbenzthiazoline-6-sulphonate) with a concentration 1 mM - 6 mM, or 5-aminosalicylic acid with a concentration 1 mM - 10 mM, or diaminobenzidine with a concentration 1 mM -10 mM.

4. The method of quantitative determination of sarcosine as per Claim 2 to 3, **which is characterised by the fact that** gold nanoparticles are prepared at 20 ° C, and their size is from 20 nm to 30 nm.

5. The method of quantitative determination of sarcosine as per Claim 2 to 4, **which is characterised by the fact that** primary anti-sarcosine antibody is fixed on the carrier, a sample containing sarcosine is added, then secondary anti-sarcosine antibody modified with gold nanoparticles is added, then auric acid is added, hydroxylamine hydrochloride and chromogenous substrate are added, and the intensity of resulting colour is evaluated.

6. The method of quantitative determination of sarcosine as per Claim 2 to 4, **which is characterised by the fact that** primary anti-sarcosine antibody is fixed on the carrier, a sample containing sarcosine is added, then sarcosine modified with gold nanoparticles is added, and after adding auric acid, hydroxylamine hydrochloride and chromogenous substrate, the intensity of resulting colour is evaluated.

7. The method of quantitative determination of sarcosine as per Claim 2 to 4, **which is characterised by the fact that** antibody against the primary anti-sarcosine antibody is fixed on the carrier, then a sample containing sarcosine is added, then primary antibody against sarcosine in the sample is added, then secondary anti-sarcosine antibody modified with gold nanoparticles is added, and after adding auric acid, hydroxylamine hydrochloride and chromogenous substrate, the intensity of resulting colour is evaluated.

8. The method of quantitative determination of sarcosine in a biological sample by means of anti-sarcosine antibodies and quantum dots as per Claim 1, **which is characterised by the fact that** primary antibody against sarcosine is fixed on the carrier, a sample containing sarcosine is added, then secondary anti-sarcosine antibody modified with quantum dots is added, and after 1 hour incubation at 37 ° C in the dark, the fluorescence intensity is evaluated.

9. The method of quantitative determination of sarcosine in a biological sample by means of anti-sarcosine antibodies and quantum dots as per Claim 1, **which is characterised by the fact that** primary anti-sarcosine antibody is fixed on the carrier, a sample containing sarcosine is added, then sarcosine modified with quantum dots is added, and after a one-hour incubation at 37°C in the dark, the fluorescence intensity is evaluated.

10. The method of quantitative determination of sarcosine in a biological sample by means of anti-sarcosine antibodies and quantum dots as per Claim 1, **which is characterised by the fact that** an antibody against the primary anti-sarcosine antibody is fixed on the carrier, then a sample containing sarcosine is added, then a primary antibody against sarcosine contained in the sample is added, then secondary anti-sarcosine antibody modified with quantum dots is added, and after a one-hour incubation at 37°C in the dark, the fluorescence intensity is evaluated.

11. The method of quantitative determination of sarcosine as per Claims 2 to 10, **which is characterised by the fact that** the carrier is a microtitration plate or magnetic particles.

12. The method of quantitative determination of sarcosine as per Claims 2 to 11, **which is characterised by the fact that** the biological sample is urine, plasma, serum or sperm.

13. The diagnostic strip (1) for the determination of sarcosine in a biological sample by means of anti-sarcosine antibody labelled with peroxidase-active gold nanoparticles or quantum dots, **which is characterised by the fact that** it is composed of a rigid pad which on one end is provided with sample zone (V) composed of the first zone (F1) of glass fibres, on which zone (E) is applied containing primary anti-sarcosine antibody labelled with gold nanoparticles or quantum dots, and of the second zone (F2) of glass fibres applied on zone (E), where zone (E) overlaps lengthwise both the first zone (F1) and the second zone (F2); towards the other end of the strip, zone (E) is followed by zone (D), on which, transversely to the strip (1), control zone (2) is applied containing antibody against anti-primary anti-sarcosine antibody, and testing zone (3) containing secondary anti-sarcosine antibody, and membrane (D) is followed by absorption zone (C) which creates the other end of the strip.

14. The diagnostic strip (1) as per Claim 13, **which is characterised by the fact that** its length (B) is 6.0 cm and width (A) is 0.5 cm, sample zone (V) is 2.0 cm long, the first zone (F1) and the second zone (F2) are 2.0 cm long each, zone (E) is 0.3 cm long, membrane (D) is 2.2 cm long and it contains a control zone (2) which is applied onto it, which, at a distance of 5.0 mm, is followed by testing zone (3) and absorption zone (C), and it is 1.5 cm long.

15. The method of sarcosine determination in a biological sample using the diagnostic strip (1) as per Claims 13 to 14, that contains primary anti-sarcosine antibody labelled with gold particles in zone (E), **which is characterised by** dipping the strip (1) in developer solution with an addition of the sample, and where developer sample contains NaCl with a concentration 0.09 M - 0.20 M, KCl with a concentration 2 mM - 5 mM, Na₂HPO₄ with a concentration 5 mM - 10 mM, KH₂PO₄ with a concentration 1 mM - 3 mM, bovine serum albumin with a concentration 0.2 % - 0,8 %, polyoxyethylene(20)-sorbitan-monolaurate with a concentration 0.5% - 2%, addition of 1 mM - 10 mM of auric acid, and 1 mM - 20 mM of hydroxylamine hydrochloride in a ration 1:1, and within 15 min, the intensity of the testing zone (3) colour is evaluated, being the sarcosine detection limit from 0.05 µM to 1.00 µM.

16. The method of sarcosine determination in a biological sample as per Claim 15, **which is characterised by the fact that** gold nanoparticles are prepared at 20 ° C, 40 ° C, 60 ° C, 80 ° C or 100 ° C, their size is from 17 nm to 37 nm and peroxidase activity is from 0.75 mU/ml to 0.92 mU/ml.

17. The method of sarcosine determination in a biological sample as per Claim 16, **which is characterised by the fact that** gold nanoparticles are prepared at 20 ° C and their size is from 20 nm to 30 nm.

18. The method of sarcosine determination in a biological sample using diagnostic strip (1) as per Claims 13 to 14, which in zone (E) contains primary antisarcosine antibody labelled with quantum dots, and **which is characterised by the fact that** sample zone (V) of strip (1) is dipped in developer solution with added sample, and where developer solution contains NaCl with a concentration 0.09 M - 0.20 M, KCl with a concentration 2 mM - 5 mM, Na₂HPO₄ with a concentration 5 mM - 10 mM, KH₂PO₄ with a concentration 1 mM - 3 mM, bovine serum albumin with a concentration 0.2 % - 0.8 %, polyoxyethylene(20)-sorbitan-monolaurate with a concentration 0.5 % - 2.0 %, and within 15 min, the intensity of the fluorescence of testing zone (3) is evaluated, being the sarcosine detection limit from 0.05 µM to 1.00 µM.

19. The method of sarcosine determination in a biological sample as per Claims 15 to 18, **which is characterised by the fact that** the biological sample is urine containing from 5 mM to 10 mM af creatinine.

20. The method of sarcosine determination in a biological sample as per Claims 15 to 18, **which is characterised by the fact that** the biological sample is plasma, serum or sperm.

## Amended claims

### Amended claims in accordance with Rule 137(2) EPC.

1. The method of quantitative determination of sarcosine in a biological sample by means of anti-sarcosine antibodies and peroxidase-like aktivity gold nanoparticles, or by means of anti-sarcosine antibodies and quantum dots, **which is characterised by the fact that** the anti-sarcosine antibodies consist of AntiSar 13 antibody anti - Sar-ebes-ebes-ebes-Cys-KLH sarcosine antigen and/or AntiSar 14 antibody anti - Sar-Aca-Cys-KLH sarcosine antigen and/or AntiSar 15 antibody anti - Me-Asp-Aca-Cys-KLH sarcosine antigen and/or AntiSar 16 antibody anti - Ser16-(Lys)8-(Lys)4-(Lys)2-Lys-β-Ala sarcosine antigen and/or AntiSar 17 antibody anti - Sar-KLH sarcosine antigen.

2. The method of quantitative determination of sarcosine by means of anti-sarcosine antibodies and peroxidase-like aktivity gold nanoparticles as per Claim 1, **which is characterised by the fact that** gold nanoparticles are prepared at 20 ° C, 40 ° C, 60 ° C, 80 ° C or 100 ° C, with a size from 17 nm to 37 nm, with peroxidase activity from 0.75 mU/ml to 0.92 mU/ml, where the detection of the sarcosine level is performed using chromogenous substrate which contains 0.5 % - 5.0 % of hydrogen peroxide and an addition of 1 mM -10 mM of auric acid and 1 mM-20 mM of hydroxylamine hydrochloride in a ratio 1:1.

3. The method of quantitative determination of sarcosine as per Claim 2, **which is characterised by the fact that** the chromogenous substrate is 3,3',5,5'-tetramethylbenzidine with a concentration from 0.2 mM - 1.0 mM or 4- minoantipyrine with a concentration 10 mM -50 mM with a sodium salt 3-(N-ethyl-3-methylaniline) of propane sulphonic acid with a concentration 0.1 mM - 5.0 mM, or O-fenylendiamine with a concentration 0.5 mM - 20.0 mM, or 2,2'-azino-bis(3-ethylbenzthiazoline-6-sulphonate) with a concentration 1 mM- 6mM, or 5-aminosalicylic acid with a concentration 1 mM - 10 mM, or diaminobenzidine with a concentration 1mM - 10 mM.

4. The method of quantitative determination of sarcosine as per Claim 2 to 3, **which is characterised by the fact that** gold nanoparticles are prepared at 20 ° C, and their size is from 20 nm to 30 nm.

5. The method of quantitative determination of sarcosine as per Claim 2 to 4, **which is characterised by the fact that** primary anti-sarcosine antibody is fixed on the carrier, a sample containing sarcosine is added, then secondary anti-sarcosine antibody modified with gold nanoparticles is added, then auric acid is added, hydroxylamine hydrochloride and chromogenous substrate are added, and the intensity of resulting colour is evaluated.

6. The method of quantitative determination of sarcosine as per Claim 2 to 4, **which is characterised by the fact that** primary anti-sarcosine antibody is fixed on the carrier, a sample containing sarcosine is added, then sarcosine modified with gold nanoparticles is added, and after adding auric acid, hydroxylamine hydrochloride and chromogenous substrate, the intensity of resulting colour is evaluated.

7. The method of quantitative determination of sarcosine as per Claim 2 to 4, **which is characterised by the fact that** antibody against the primary anti-sarcosine antibody is fixed on the carrier, then a sample containing sarcosine is added, then primary antibody against sarcosine in the sample is added, then secondary anti-sarcosine antibody modified with gold nanoparticles is added, and after adding auric acid, hydroxylamine hydrochloride and chromogenous substrate, the intensity of resulting colour is evaluated.

8. The method of quantitative determination of sarcosine in a biological sample by means of anti-sarcosine antibodies and quantum dots as per Claim 1, **which is characterised by the fact that** primary antibody against sarcosine is fixed on the carrier, a sample containing sarcosine is added, then secondary anti-sarcosine antibody modified with quantum dots is added, and after 1 hour incubation at 37 ° C in the dark, the fluorescence intensity is evaluated.

9. The method of quantitative determination of sarcosine in a biological sample by means of anti-sarcosine antibodies and quantum dots as per Claim 1, **which is characterised by the fact that** primary anti-sarcosine antibody is fixed on the carrier, a sample containing sarcosine is added, then sarcosine modified with quantum dots is added, and after a one-hour incubation at 37°C in the dark, the fluorescence intensity is evaluated.

10. The method of quantitative determination of sarcosine in a biological sample by means of anti-sarcosine antibodies and quantum dots as per Claim 1, **which is characterised by the fact that** an antibody against the primary anti-sarcosine antibody is fixed on the carrier, then a sample containing sarcosine is added, then a primary antibody against sarcosine contained in the sample is added, then secondary anti-sarcosine antibody modified with quantum dots is added, and after a one-hour incubation at 37°C in the dark, the fluorescence intensity is evaluated.

11. The method of quantitative determination of sarcosine as per Claims 2 to 10, **which is characterised by the fact that** the carrier is a microtitration plate or magnetic particles.

12. The method of quantitative determination of sarcosine as per Claims 2 to 11, **which is characterised by the fact that** the biological sample is urine, plasma, serum or sperm.

13. The method of sarcosine determination in a biological sample as per Claim 1 using the diagnostic strip (1) that it is composed of a rigid pad which on one end is provided with sample zone (V) composed of the first zone (F1) of glass fibres, on which zone (E) is applied containing primary anti-sarcosine antibody labelled with peroxidase-like aktivity gold nanoparticles and of the second zone (F2) of glass fibres applied on zone (E), where zone (E) overlaps lengthwise both the first zone (F1) and the second zone (F2); towards the other end of the strip, zone (E) is followed by zone (D), on which, transversely to the strip (1), control zone (2) is applied containing antibody against anti-primary anti-sarcosine antibody, and testing zone (3) containing secondary anti-sarcosine antibody, and membrane (D) is followed by absorption zone (C) which creates the other end of the strip, **which is characterised by** dipping the strip(1) in developer solution with an addition of the sample, and where developer sample contains NaCl with a concentration 0.09 M - 0.20 M, KCl with a concentration 2 mM - 5 mM, Na₂HPO₄ with a concentration 5 mM - 10 mM, KH₂PO₄ with a concentration 1 mM - 3 mM, bovine serum albumin with a concentration 0.2 % - 0,8 %, polyoxyethylene(20)-sorbitan-monolaurate with a concentration 0.5% - 2%, addition of 1 mM - 10 mM of auric acid, and 1 mM - 20 mM of hydroxylamine hydrochloride in a ration 1:1, and within 15 min, the intensity of the testing zone (3) colour is evaluated.

14. The method of sarcosine determination in a biological sample as per Claim 13, **which is characterised by the fact that** gold nanoparticles are prepared at 20 ° C, 40 ° C, 60 ° C, 80 ° C or 100 ° C, their size is from 17 nm to 37 nm and peroxidase activity is from 0.75 mU/ml to 0.92 mU/ml.

15. The method of sarcosine determination in a biological sample as per Claim 14, **which is characterised by the fact that** gold nanoparticles are prepared at 20 ° C and their size is from 20 nm to 30 nm.

16. The method of sarcosine determination in a biological sample using the diagnostic strip (1) as per Claim 1, that it is composed of a rigid pad which on one end is provided with sample zone (V) composed of the first zone (F1) of glass fibres, on which zone (E) is applied containing primary anti-sarcosine antibody labelled with quantum dots and of the second zone (F2) of glass fibres applied on zone (E), where zone (E) overlaps lengthwise both the first zone (F1) and the second zone (F2); towards the other end of the strip, zone (E) is followed by zone (D), on which, transversely to the strip (1), control zone (2) is applied containing antibody against anti-primary anti-sarcosine antibody, and testing zone (3) containing secondary anti-sarcosine antibody, and membrane (D) is followed by absorption zone (C) which creates the other end of the strip, **which is characterised by** dipping the strip(1) in developer solution with an addition of the sample, and where developer sample contains NaCl with a concentration 0.09 M - 0.20 M, KCl with a concentration 2 mM - 5 mM, Na₂HPO₄ with a concentration 5 mM - 10 mM, KH₂PO₄ with a concentration 1 mM - 3 mM, bovine serum albumin with a concentration 0.2 % - 0,8 %, polyoxyethylene(20)-sorbitan-monolauratewith a concentration 0.5% - 2%, and within 15 min, the intensity of the fluorescence oftesting zone (3) is evaluated.

17. The method of sarcosine determination in a biological sample as per Claims 13 to 16, **which is characterised by the fact that** the biological sample is urine containing from 5 mM to 10 mM of creatinine.

18. The method of sarcosine determination in a biological sample as per Claims 13 to 16, **which is characterised by the fact that** the biological sample is plasma, serum or sperm.

19. The diagnostic strip (1) suitable for conducting the method as per Claims 13 to 16, **which is characterised by the fact that** it is composed of a rigid pad which on one end is provided with sample zone (V) composed of the first zone (F1) of glass fibres, on which zone (E) is applied containing primary anti-sarcosine antibody labelled with quantum dots and of the second zone (F2) of glass fibres applied on zone (E), where zone (E) overlaps lengthwise both the first zone (F1) and the second zone (F2); towards the other end of the strip, zone (E) is followed by zone (D), on which, transversely to the strip (1), control zone (2) is applied containing antibody against anti-primary anti-sarcosine antibody, and testing zone (3) containing secondary anti-sarcosine antibody, and membrane (D) is followed by absorption zone (C) which creates the other end of the strip.

20. The diagnostic strip (1) as per Claim 19, **which is characterised by the fact that** its length (B) is 6.0 cm and width (A) is 0.5 cm, sample zone (V) is 2.0 cm long, the first zone (F1) and the second zone (F2) are 2.0 cm long each, zone (E) is 0.3 cm long, membrane (D) is 2.2 cm long and it contains a control zone (2) which is applied onto it, which, at a distance of 5.0 mm, is followed by testing zone (3) and absorption zone (C), and it is 1.5 cm long.
